# EUROPÄISCHE PATENTSCHRIFT

(11) **EP 0 683 820 B1**
(45) Veröffentlichungstag und Bekanntmachung des Hinweises auf die Patenterteilung: **21.07.1999**
(21) Anmeldenummer: 94906198.0
(22) Anmeldetag: 02.02.1994
(51) Int. Cl.: C12P 9/00, C12P 19/02

(54) **ENZYMATISCHES VERFAHREN ZUR HERSTELLUNG VON DIHYDROXYACETONPHOSPHAT AUS GLYCERINPHOSPHAT UND SEINE VERWENDUNG IN ENZYMATISCHEN ALDOLADDITIONEN**
ENZYMATIC PROCESS FOR PRODUCING DIHYDROXYACETONE PHOSPHATE FROM GLYCERINE PHOSPHATE AND ITS USE IN ENZYMATIC ALDOL ADDITIONS
PROCEDE ENZYMATIQUE POUR FABRIQUER DU DIHYDROXYACETONE PHOSPHATE A PARTIR DE GLYCEROPHOSPHATE ET SON UTILISATION EN ADDITIONS ALDOLIQUES ENZYMATIQUES

(30) Priorität: 11.02.1993 DE 4304097
(43) Veröffentlichungstag der Anmeldung: 29.11.1995
(73) Patentinhaber: Roche Diagnostics GmbH, 68298 Mannheim (DE)
(72) Erfinder: FESSNER, Wolf-Dieter, D-79194 Gundelfingen (DE)
(86) Internationale Anmeldenummer: EP9400291
(87) Internationale Veröffentlichungsnummer: WO9418338

(56) Entgegenhaltungen:
- EP-A- 0 255 334
- DE-C- 4 111 971
- US-A- 4 440 854

## Beschreibung

Die Erfindung betrifft ein Verfahren zur Herstellung von Dihydroxyacetonphosphat (DHAP) durch enzymatische Oxidation von Glycerinphosphat sowie dessen Verwendung in situ durch Umsetzung in einer gekoppelten enzymatischen Aldoladdition.

Die stereoselektive asymmetrische Synthese polyhydroxylierter Verbindungen ist von aktuellem Interesse wegen deren Bedeutung als Komponenten oder Vorstufen pharmazeutischer Wirkstoffe wie Antibiotika oder Glykosidase-Inhibitoren. Eine wertvolle Methodik stellen enzymatische Aldoladditionen dar, da sich damit unter milden Reaktionsbedingungen alle vier möglichen diastereomeren Ketosen und bestimmte Derivate hoch diastereoselektiv und damit in hoher chemischer und optischer Reinheit herstellen lassen (DE 41 11 971 ). Während die entsprechenden Enzyme, die DHAP-Aldolasen, eine Vielfalt von Aldehyden als elektrophile Komponente akzeptieren, benötigen diese Enzyme daruber hinaus Dihydroxyacetonphosphat (DHAP) als essentielles Aldoldonor-Substrat.

Kommerzielles DHAP oder direkte Acetalvorstufen kommen als Edukt für technische Synthesen aus wirtschaftlichen Gründen nicht in Betracht. Zudem ist freies DHAP relativ instabil und zerfällt in Methylglyoxal, das Enzyme irreversibel desaktiviert, und in anorganisches Phosphat, das die Aldolasen inhibiert. Obwohl verschiedene Verfahren zur chemischen oder enzymatischen Synthese von DHAP bekannt sind, weist jedes einzelne nach Aufwand und Produktqualität gravierende Nachteile auf:
- Die chemische Phosphorylierung von Dihydroxyaceton mit Phosphoroxychlorid in Acetonitril/Pyridin liefert in schlechter Ausbeute (60%, nach mehreren Berichten nicht reproduzierbar) ein Produkt, das stark mit anorganischem Phosphat verunreinigt ist (C.-H. Wong und G.M. Whitesides, J. Org. Chem. 1983, 48, 3199).
- Das Dimethoxyacetal, aus dem DHAP durch saure Hydrolyse freigesetzt wird, erfordert eine aufwendige Herstellung aus 3-Chlor-1,2-propandiol (8 Synthesestufen, Gesamtausbeute ca. 17%; C.E. Ballou und H.O.L Fischer, J. Am. Chem. Soc. 1956, 78, 1659).
- Das Diethylacetal des Dimeren kann unter erheblichem Sach- und Zeitaufwand (3-5 Synthesestufen) nach drei Varianten chemisch phosphoryliert werden z.B. mit Diphenylchlorphosphat (R.L. Colbran et al., Carbohydr. Res. 1967, 4, 355), Phosphoroxychlorid (F. Effenberger und Straub, Tetrahedron Lett. 1987, 28, 1641) oder Dibenzyl-N,N-diethylphosphoramidit (R.L. Pederson et al. Tetrahedron, 1991, 47, 2643). DHAP, das durch saure Hydrolyse aus dieser Vorstufe erzeugt wird, ist durch ca. 20% anorganisches Phosphat verunreinigt und wird lediglich in einer Gesamtausbeute von 50 % erhalten. Entsprechend hergestelltes DHAP ist somit nachteilig für bestimmte Folgeverfahren, wie z.B. zur Herstellung von Ketosen durch enzymatische Umsetzung von Aldehyden mit DHAP in Gegenwart von geeigneten Aldelosen (DE 4111971).
- Enzymatische Phosphorylierung von Dihydroxyaceton durch Glycerinkinase-ATP (D.C. Crans und G.M. Whitesides, J. Am. Chem. Soc. 1985, 107, 7019) liefert eine DHAP-Qualität, die durch starke Verunreinigung mit Acetat und Phosphat gekennzeichnet ist, da das zur Cofaktorregenerierung verwendete Acetylphosphat hydrolytisch instabil ist. Das Enzym wird durch das stark elektrophile DHAP in beträchtlichem Ausmaß irreversibel inaktiviert und kann auch durch Immobilisierung nur begrenzt stabilisiert werden.
- Die enzymatische Spaltung von Fructose-1,6-bisphosphat mit Fructose-1,6-bisphosphat-Aldolase verwendet ein vergleichsweise aufwendiges Ausgangsmaterial, liefert nur eine niedrige Gleichgewichtskonzentration an DHAP und unterliegt der Beschränkung auf Synthesereaktionen mit derselben Aldolase (O. Meyerhof und K. Lohmann, Biochem. Z. 1934, 271, 89; US 4.440.854; W.-D. Fessner und C. Walter, Angew. Chem. 1992, 104, 643).
- Die enzymatische Umsetzung von Glycerin mittels Glycerinkinase und Glycerinphosphatoxidase führt lediglich zu DHAP in nicht-isolierbaren Mengen in nano-bis mikromolarem Maßstab (JP 03/228 699, FR 2 620 732, US 4.784.945, DE 3 731 876, EP 0 255 334, EP 0 354 551).

Aufgabe der Erfindung ist daher Dihydroxyacetonphosphat aus leicht zugänglichen Ausgangsstoffen in hoher chemischer Reinheit und Ausbeute herzustellen, und dabei gleichzeitig durch Enzymkatalyse unter so milden Reaktionsbedingungen zu arbeiten, daß das labile Produkt möglichst in situ in einer gekoppelten Reaktion durch alle Arten von DHAP-Aldolasen umgesetzt werden kann.

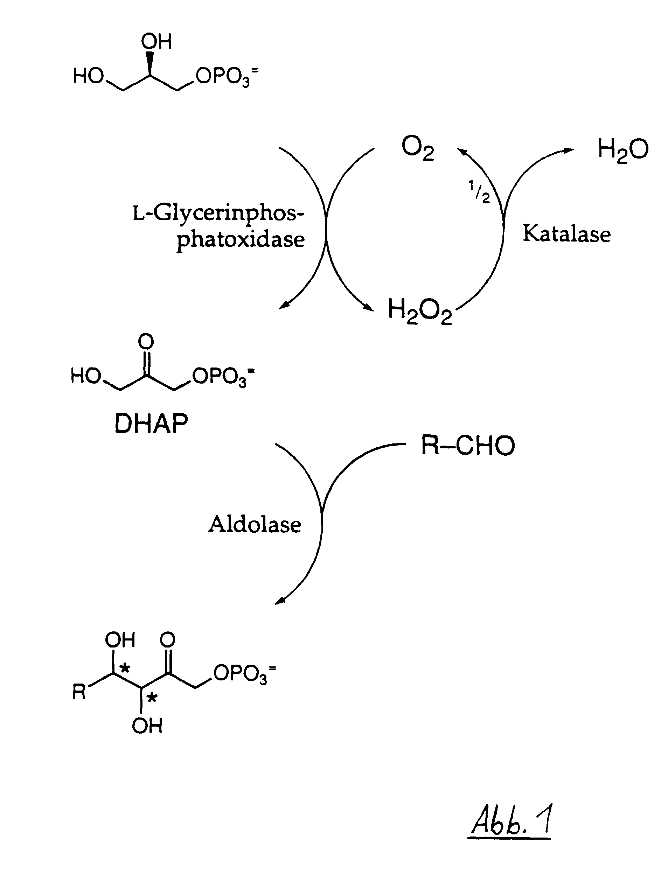

Die Aufgabe wird gelöst durch ein Verfahren zur Herstellung von DHAP, dadurch gekennzeichnet, daß Glycerin-monophosphat mit einer Glycerinphosphatoxidase (GPO) [EC 1.1.3.21] in Gegenwart von Sauerstoff, Luft oder Wasserstoffperoxid und überschüssigen Mengen eines H₂O₂-zersetzenden Enzyms wie beispielsweise Katalase [EC 1.11.1.6] oxidiert wird. In Abhängigkeit von der spezifischen Enzymaktivität der jeweils verwendeten Enzympräparation und bedingt durch die Grenzen der Enzymlöslichkeiten, werden die Enzyme vorzugsweise in folgenden Konzentrationsbereichen verwendet: GPO 1 - 1000 U/ml und Katalase 5 - 1 000 000 U/ml, wobei die Katalaseaktivität das ein- bis tausendfache der GPO-Aktivität erreichen sollte. Mit löslichen Enzymen wird besonders bevorzugt mit 1 - 10 U/ml GPO und dem 5 - 100fachen an Katalaseaktivitat gearbeitet. Für entsprechende Abwandlungen (z.B. bei Anwendung von Enzym-Membranreaktoren oder Enzym-Immobilisaten) können Abweichungen hiervon erforderlich werden, die sich jedoch für den Fachmann auf der Basis der hier gegebenen Lehre durch gängige Versuche finden lassen. Weiter wird vorzugsweise unter Sauerstoffatmosphäre und erhöhtem Druck (1-5 bar (∼1-5 atm)) gearbeitet. Die Reaktionstemperatur kann zwischen -10°C und 60°C, bevorzugt zwischen 0°C und 40°C variiert werden, und ganz besonders bevorzugt wird bei hinreichender Sauerstofflöslichkeit bei 20° bis 25°C gearbeitet. Alternativ kann der benötigte Sauerstoff auch durch Zudosieren einer wässrigen Lösung von Wasserstoffperoxid (H₂O₂) nach Abbildung 1 erzeugt werden. Dadurch wird überraschenderweise ein von weiteren Komponenten freies DHAP-Produkt erhalten.

DHAP wirkt in höheren Konzentrationen als kompetitiver Inhibitor auf die Glycerinphosphatoxidase, so daß die Reaktion zwischen 60 und 95 % Umsatz, in der Regal jedoch bei ungefähr 90 % beendet wird. Dies bedeutet, daß das nach diesem Verfahren erzeugte Produkt je nach Reaktionsführung zwischen 40 und 5 % Glycerinphosphat enthalten kann.

Zur Lagerung kann das DHAP in Form der Produktlösung bei - 80°C eingefroren werden oder in eine lagerstabile Form überführt werden. Hierzu dient beispielsweise die Fällung als Bariumsalz (D.C. Crans und G.M. Whitesides, J. Am. Chem. Soc. 1985, 107, 7019) oder das vollständige Konzentrieren der Lösung unter schonenden Bedingungen (verminderter Druck, Kälte), worauf das Produkt als festes Salz erhalten wird. In letzerem Fall kann durch Wahl des mit dem Glycerinphosphat eingebrachten Kations (z.B. Alkalimetallionen) bereits die gewünschte Salzform des DHAP vorbestimmt werden.

Das erfindungsgemäße Verfahren zur oxidativen DHAP-Herstellung läßt sich problemlos unter Zugabe eines beliebigen Aldehyds mit einer enzymatischen Aldoladdition koppeln (Abbildung 1). Dieser Befund ist ebenso überraschend, da nicht zu erwarten war, daß die maximal erreichte Konzentration an Wasserstoffperoxid nicht zur Desaktivierung der DHAP-Aldolasen führt. Selbst bei der Verwendung von sauerstoffgesättigten Lösungen und unter erhöhtem Sauerstoffpartialdruck bis zu 5 bar (~5 atm) Überdruck wurden keine nachteiligen Einflüsse auf die Enzymstabilitäten festgestellt. Die Möglichkeit zur Kopplung der nebenproduktfrei DHAP-produzierenden und einer DHAP-verbrauchenden Reaktion vermeidet nicht nur die Produktinhibition der Glycerinphosphatoxidase und ermöglicht damit einen quantitativen Umsatz von L-Glycerin-3-phosphat, sondern unterdrückt auch nahezu völlig die Zersetzung des labilen DHAP, da dieses nicht akkumulieren kann. Als mögliche Aldolasen kommen beispielsweise Fructose-1,6-biphosphataldolase [EC 4.1.2.13], Tagatose-1,6-biphosphataldolase [EC 4.1.2.-], Fuculose-1-phosphataldolase [EC 4.1.2.17] oder Rhamnulose-1-phosphataldolase [EC 4.1.2.19] in betracht. Die gekoppelten Reaktionen werden in wässrigem Milieu bei schwach saurem pH-Wert (ca. 5.5 - 8.0, vorzugsweise 6.5 - 7.0) durchgeführt. Dadurch ist eine ausreichende Stabilität der Reaktionskomponente DHAP und eine hohe enzymatisch katalysierte Reaktionsgeschwindigkeit gewährleistet.

Die Aldoladdukte sind ebenfalls vorteilhaft durch Konzentrieren der Lösung in sehr hoher Ausbeute in reiner Form als kristalline Salze erhältlich, wobei das Gegenkation mit der Form des Glycerinphosphatsalzes wahlbar ist. Das wäßrige Milieu kann dabei gegebenenfalls zur Verbesserung der Löslichkeit lipophiler Substrate bis zu 50 Vol-% organisches Cosolvens wie zum Beispiel niedere aliphatische Alkohole (Methanol, Ethanol, n- oder i-Propanol), Dimethylsulfoxid, Dimethylformamid oder Acetonitril enthalten. Zu berücksichtigen ist hierbei jedoch, daß die Enzymaktivitäten durch die Anwesenheit organischer Lösungsmittel vermindert werden können, daher wird vorzugsweise ohne bzw. mit maximal 30 Vol-% Cosolvens gearbeitet.

Das erfindungsgemäße Verfahren ist insbesondere auch für die Herstellung isotopenmarkierter Zucker und ihrer Derivate in vorteilhafter Weise anwendbar, da die wertvollen markierten Glycerinvorstufen nahezu ohne Substanzverlust in Form markierten DHAPs oder des durch Isomerisierung mit Triosephosphatisomerase [EC 5.3.1.1] erzeugbaren, markierten D-Glycerinaldehyd-3-phosphats in die entsprechenden Aldolprodukte überführbar sind. Beispielsweise kommen ²H, ³H, ¹²C, ¹³C, ¹⁷O und ³²P für eine solche spezifische Markierung in betracht.

Der Einsatz löslicher Enzyme erleichtert die Dosierung und Bestimmung der Restaktivitäten, jedoch kann eine Immobilisierung an feste Träger, z.B. an Eupergit® C, die Stabilität der Enzyme ebenfalls vorteilhaft erhöhen.

Im Rahmen des erfindungsgemäßen Verfahrens können anstelle von enantiomerenreinem L-Glycerin-3-phosphat auch diese Substanz als Komponente enthaltende Gemische wie racemisches DL-Glycerinphosphat oder Gemische der Glycerin-1(2)-monophosphate eingesetzt werden, ohne daß eine merkliche Einbuße der Umsatzgeschwindigkeit auftritt. Dies ist für den Fachmann uberraschend, da durch für die entsprechenden Enzyme nicht spezifischen Isomeren die Enzyme oft inhibiert werden.

Zur Reinigung der Aldolprodukte aus den gekoppelten Synthesen kann nach Phosphatesterhydrolyse (z.B. durch Phosphatasen) das aus den nicht umgesetzten Glycerinphosphat-Isomeren resultierende Glycerin leicht durch Überführung in das flüchtige Acetonacetal und Erwärmen unter Vakuum abgetrennt werden.

Für das erfindungsgemäße Verfahren sind kommerziell erhältliche Glycerinphosphatoxidasen aus verschiedenen Organismen geeignet (Boehringer Mannheim GmbH; Sigma Chemie GmbH, spez. Aktivität von GPO bis 200 U/mg). Im Rahmen der vorliegenden Erfindung wurde insbesondere ein Enzympräparat aus Mikroorganismen (Toyobo) verwendet.

Als Aldolasen können ebenfalls entweder die von den genannten Firmen vertrieben Enzyme oder die nach bekannten Verfahren aus den in der DE 41 11 971 genannten Bakterienstämmen isolierten Enzyme verwendet werden.

Verfahren zur Herstellung der Ausgangsverbindungen, wie L-Glycerin-3-phosphat sind bekannt. Beispielsweise kann dies durch enzymatische Phosphorylierung von Glycerin erfolgen (D.C. Crans und G.M. Whitesides, J. Am. Chem. Soc. 1985, 107, 7019). Handelsübliche Formen von isotopenmarkiertem Glycerin lassen sich ebenfalls mit dieser Technik phosphorylieren. Andere mögliche Ausgangsstoffe für das erfindungsgemäße Verfahren wie Glycerinmonophosphate mit unterschiedlichem Gehalt am L-Enantiomeren und in Form verschieden formulierter Salze sind kommenziell erhältlich.

Abbildung 1: Reaktionsschema für die enzymatische Oxidation von L-Glycerin-3-phosphat zu DHAP und Verwendung in gekoppelten Aldoladditionen.

Nachfolgende Beispiele sollen die Erfindung näher erläutern, ohne diese zu beschränken.

### Beispiel 1: Synthese von Dihydroxyacetonphosphat

Eine Lösung von 17.2g (100mnol) L-Glycerin-3-phosphorsäure in 500ml dest. Wasser wurde mit Lithiumhydroxid auf pH6.8 eingestellt und auf 1000ml verdünnt. Nach Durchleiten von Sauerstoff (15 min) zur Sättigung wurden 1000U L-Glycerin-3-phosphatoxidase (GPO) und 5000U Katalase (spez. Aktivität bis 50 000 U/mg; Sigma) zugegeben und die Lösung bei 20°C unter einem Sauerstoffdruck von ca. 2 bar (∼2 atm) mechanisch gerührt. Der Umsatz wurde durch enzymatischen Assay auf gebildetes DHAP sowie durch ¹H- und ³¹P-NMR-Spektroskopie kontrolliert. Nach beendeter Reaktion (ca. 90% Umsatz) wurde die Lösung über Aktivkohle filtriert, mit 1M Lithiumhydroxid neutralisiert und am Rotationsverdampfer (Kühlfalle mit Trockeneis/Acetonitril) bei einer Temperatur unter 20°C im Vakuum zügig bis zur Trockne eingeengt. Das Produkt fiel dabei als farbloses, festes Li-salz von hoher Reinheit an (ca. 90%; 10% Glycerinphosphat; <5% anorganisches Phosphat).

Alternativ wurde L-Glycerin-3-phosphat als Kalium- bzw. Natrium-Salz umgesetzt, wobei die entsprechenden DHAP-Salze erhalten wurden. In diesen Fällen wurden die Produktlösungen mit Kationenaustauscher Dowex® AG50W-X8 (H⁺-Form; Bio-Rad) auf pH 3-4 eingestellt und bei -78°C eingefroren.

### Beispiel 2: Synthese von [2,5-¹³C₂]-D-Fructose-1,6-biphosphat

Eine Lösung von [2-¹³C]-L-Glycerin-3-phosphat (Biscyclohexylammoniumsalz; 370 mg, 1.0 mmol) in 10 ml Wasser wurde bei pH 6.8 mit 50 U GPO, 1000 U Katalase, 50 U Fructose-1,6-bisphosphataldolase (aus E. coli, [EC 4.1.2.13]) und 50 U Triosephosphatisomerase ([EC 5.3.1.1]) versetzt und bei 25°C unter Sauerstoffatmosphäre mechanisch gerührt bis die DC-Kontrolle quantitativen Umsatz zeigte. Nach Neutralisieren mit Cyclohexylamin, Konzentrieren auf ca. 3 ml und Verdünnen mit Ethanol kristallisierte reines [2,5-¹³C₂]-D-Fructose-1,6-bisphosphat als Tetra(cyclohexylammonium)salz. Ausbeute 350 mg (95% d.Th.).

### Beispiel 3: Synthese von D-Xylulose-1-phosphat

Eine Lösung von L-Glycerin-3-phosphat (Kaliumsalz; 1.0 mmol) in 10 ml Wasser wurde bei pH 6.8 mit 70 U GPO und 1000 U Katalase versetzt und bei 25°C im offenen Erlenmeyerkolben bei 100 Upm geschwenkt. Bei ca. 60 % Umsatz laut enzymatischem Assay auf DHAP wurden eine Lösung von Glykolaldehyd (72 mg, 1.2 mmol) in 1.0 ml Wasser und 50 U Fructose-1,6-bisphosphataldolase (aus Kaninchenmuskel, [EC 4.1.2.13]) zugegeben und die Reaktionslö-sung weiter geschwenkt. Nach vollständigem Umsatz von Glycerinphosphat bzw. DHAP (DC-, ¹H-NMR-Kontrolle) wurde das Produkt mittels Ionenaustauschchromatographie an Anionenaustauscher AG1-X8, (HCO₃⁻-Forn, 4 ml) durch Elution mit Triethylammoniumhydrogencarbonat isoliert. Nach Überführung in das Cyclohexylammoniumsalz erhielt man D-Xylulose-1-phosphat als farblosen Feststoff. Chemische Ausbeute 410 mg (96 % d.Th.).

### Beispiel 4: Synthese von D-Ribulose-1-phosphat

Eine Lösung von L-Glycerin-3-phosphat (Biscyclohexylammoniumsalz; 370 mg, 1.0 mmol) in 10 ml sauerstoffgesättigtem Wasser wurde bei pH 6.8 mit 70 U GPO und 1000 U Katalase versetzt und im 50 ml Hydrierkolben unter positivem Sauerstoffdruck bei 100 Upm geschwenkt. Analog zu Beispiel 2 wurden Glykolaldehyd und 50 U Fuculose-l-phosphataldolase (aus E. coli, [EC 4.1.2.17]) zugegeben und nach beendeter Reaktion das D-Ribulose-1-phosphat als festes, farbloses Cyclohexylammoniumsalz isoliert. Chemische Ausbeute: 396 mg (93 % d.Th.).

### Beispiel 5: Synthese von L-Fructose 1-phosphat

Eine Lösung von L-Glycerin-3-phosphat (Biscyclohexylammoniumsalz; 370 mg, 1.0 mmol) und L-Glycerinaldehyd (110 mg, 1.2 mmol) in 10 ml sauerstoffgesättigtem Wasser wurde bei pH 6.8 mit 70 U GPO, 1000 U Katalase und 50 U Rhamnulose-1-phosphataldolase (aus E. coli, [EC 4.1.2.19]) versetzt und bis zum vollständigen Umsatz unter Sauerstoffatmosphäre bei 100 Upm geschwenkt. Nach Filtrieren uber Aktivkohle wurde mit 1.0 M ethanolischer Cyclohexylaminlosung auf pH 7.5 eingestellt und am Rotationsverdampfer zur Trockene eingeengt. Der feste Rückstand wurde in 0.5 ml Wasser aufgenommen, filtriert und mit 2.5 ml trockenem Ethanol und soviel trockenem Aceton versetzt, bis eine leichte Trübung bestehen blieb. Kristallisation bei 4°C ergab L-Fructose-1-phosphat als Cyclohexylammoniumsalz in Form farbloser Nadeln. Chemische Ausbeute 370 mg (85 % d.Th.).

### Beispiel 6: Synthese von L-Fructose

Eine sauerstoffgesättigte Lösung von DL-glycerin-1-phosphat (Natriumsalz, Hexahydrat; 1.62 g, 5.0 mmol) in 25 ml Wasser wurde mit 200 U GPO und 1000 U Katalase versetzt und unter einem Sauerstoffdruck von 2 atm mechanisch geruhrt bis die Reaktionslösung ca. 2 mmol DHAP enthielt. Dann wurden L-Glycerinaldehyd (270 mg, 3.0 mmol) sowie 50 U Rhamnulose-1-phosphataldolase (aus E. coli, [EC 4.1.2.19]) zugegeben und die Reaktionslösung bis zum vollständigen Umsatz gerührt. Nach Filtrieren über Aktivkohle wurde mit Ionenaustauscher (AG50W-X8, H⁺-Form) auf pH 5 eingestellt und mit 100 U saurer Phosphatase versetzt. Nach vollständiger Hydrolyse der Phosphatester (DC-Kontrolle) wurde die Lösung durch Filtrieren über 20 ml Kationenaustauscher (AG50W-X8, H⁺-Form), dann 20 ml Anionenaustauscher (AG1-X8, HCO₃⁻-Form) entsalzt und im Vakuum konzentriert. Zur Abtrennung des Glycerins wurde der Rückstand in Aceton aufgenommen, mit einer katalytischen Menge an p-Toluolsulfonsaure versetzt und nach vollständigem Umsatz im Hochvakuum bei ca. 50°C von Losungsmittel und 1,2-Isopropylidenglycerin befreit. Nach Aufnahme des festen Rückstands in Wasser und vollständiger Hydrolyse der Zuckeracetale wurde die L-Fructose aus 80 % wässrigem Ethanol kristallisiert. Chemische Ausbeute 360 mg (80% d.Th.).

Ein analoges Experiment ausgehend von einem Gemisch der Glycerin-1(2)-monophosphate (Natriumsalz, Pentahydrat, enthielt ca. 50% DL-1-Phosphat und ca. 50% 2-Phosphat; 3.06 g, 10 mmol) lieferte nach entsprechender Aufarbeitung L-Fructose in gleicher Ausbeute.

## Patentansprüche

1. Verfahren zur enzymatischen Herstellung von Dihydroxyacetonphosphat aus Glycerinmonophosphat, dadurch gekennzeichnet, daß man in Gegenwart von molekularem Sauerstoff, Luft oder Waserstoffperoxid eine Glycerinphosphatoxidase und ein H₂O₂-zersetzendes Enzym verwendet.

2. Verfahren nach Anspruch 1, dadurch gekennzeichnet, daß man Glycerinphosphatoxidase und das ein- bis tausendfache der Glycerinphospohatoxidase-Aktivität an Aktivität für das H₂O₂-zersetzende Enzym verwendet.

3. Verfahren nach einem der Ansprüche 1 oder 2, dadurch gekennzeichnet, daß man 1 bis 1000 U/ml Glycerinphosphatoxidase und 5 bis 10⁶ U/ml des H₂O₂-zersetzenden Enzyms verwendet.

4. Verfahren nach einem der Ansprüche 1-3, dadurch gekennzeichnet, daß unter Sauerstoffatmosphäre bei einem Druck von 1-5 bar (1-5 atm), einem pH-Wert von 5,5 - 8,0 und einer Temperatur von 0° bis 40°C in Gegenwart von 0-50 % (v/v) eines organischen Lösungsmittels gearbeitet wird.

5. Verfahren nach einem der Ansprüche 1-4, dadurch gekennzeichnet, daß man als H₂O₂-zersetzendes Enzym Katalase verwendet.

6. Verfahren nach einem der Ansprüche 1-5, dadurch gekennzeichnet, daß man spezifisch markiertes Glycerinphosphat verwendet.

7. Verfahren zur Herstellung von Zuckern oder deren Derivate aus Glycerinmonophosphat, dadurch gekennzeichnet, daß das Verfahren nach einem der Ansprüche 1-6 in Gegenwart einer Aldolase und eines Aldehyds durchgeführt wird.

8. Verfahren nach Anspruch 7, dadurch gekennzeichnet, daß man eine Fructose-1,6-biphosphataldolase, Tagatose-1,6-biphosphataldolase, Fuculose-1-phosphataldolase oder Rhamnulose-1-phosphataldolase verwendet.

9. Verfahren nach einem der Ansprüche 1-8, dadurch gekennzeichnet, daß mindestens eines der Enzyme in immobilisierter Form vorliegt.

## Claims

1. Process for the enzymatic production of dihydroxyacetone phosphate from glycerol monophosphate, wherein a glycerophosphate oxidase and a H₂O₂-decomposing enzyme are used in the presence of molecular oxygen, air or hydrogen peroxide.

2. Process as claimed in claim 1, wherein one uses glycerophosphate oxidase and one-fold to one thousand-fold of the GPO activity as the activity for the H₂O₂-decomposing enzyme.

3. Process as claimed in one of the claims 1 or 2, wherein 1 to 1000 U/ml glycerophosphate oxidase and 5 to 10⁶ U/ml of the H₂O₂-decomposing enzyme are used.

4. Process as claimed in one of the claims 1-3, wherein one works under an oxygen atmosphere at a pressure of 1-5 bar (1-5 atm) at a pH value of 5.5 - 8.0 and a temperature of 0° to 40°C in the presence of 0-50 % (v/v) of an organic solvent .

5. Process as claimed in one of the claims 1-4, wherein catalase is used as the H₂O₂-decomposing enzyme.

6. Process as claimed in one of the claims 1-5, wherein specifically-labelled glycerophosphate is used.

7. Process for the production of sugars or their derivatives from glycerol monophosphate, wherein the process is carried out as claimed in one of the claims 1-6 in the presence of an aldolase and an aldehyde.

8. Process as claimed in claim 7, wherein a fructose-1,6-diphosphate aldolase, tagatose-1,6-diphosphate aldolase, fuculose-1-phosphate aldolase or rhamnulose-1-phosphate aldolase is used.

9. Process as claimed in one of the claims 1-8, wherein at least one of the enzymes is present in an immobilized form.

## Revendications

1. Procédé pour la préparation enzymatique du phosphate de dihydroxyacétone à partir du monophosphate de glycérol, caractérisé en ce qu'on utilise, en présence d'oxygène moléculaire, d'air ou de peroxyde d'hydrogène, de la glycérolphosphatoxydase et une enzyme décomposant le H₂O₂.

2. Procédé selon la revendication 1, caractérisé en ce qu'on utilise de la glycérolphosphatoxydase et une activité de la glycérolphosphatoxydase de 1 à 1000 fois supérieure à l'activité de l'enzyme décomposant le H₂O₂.

3. Procédé selon la revendication 1 ou 2, caractérisé en ce qu'on utilise de la glycérolphosphatoxydase à concurrence de 1 à 1000 U/ml et l'enzyme décomposant le H₂O₂ à concurrence de 5 à 10⁶ U/ml.

4. Procédé selon l'une quelconque des revendications 1 à 3, caractérisé en ce qu'on travaille sous atmosphère d'oxygène sous une pression de 1-5 bar (1-5 atm), à une valeur de pH de 5,5 à 8,0 et à une température de 0°C à 40°C en présence d'un solvant organique à 0-50% (volume/volume).

5. Procédé selon l'une quelconque des revendications 1 à 4, caractérisé en ce qu'on utilise de la catalase à titre d'enzyme décomposant le H₂O₂.

6. Procédé selon l'une quelconque des revendications 1 à 5, caractérisé en ce qu'on utilise du phosphate de glycérol marqué de manière spécifique.

7. Procédé pour la préparation de sucres ou de leurs dérivés à partir de monophosphate de glycérol, caractérisé en ce qu'on effectue le procédé selon l'une quelconque des revendications 1 à 6 en présence d'une aldolase et d'un aldéhyde.

8. Procédé selon la revendication 7, caractérisé en ce qu'on utilise une fructose-1,6-biphosphataldolase, une tagatose-1,6-biphosphataldolase, une fuculose-1-phosphataldolase ou une rhamnulose-1-phosphataldolase.

9. Procédé selon l'une quelconque des revendications 1 à 8, caractérisé en ce qu'au moins une des enzymes est présente sous forme immobilisée.
